(19) Europäisches Patentamt / European Patent Office / Office européen des brevets

(11) EP 2 691 478 B1

(12) # EUROPÄISCHE PATENTSCHRIFT

(45) Veröffentlichungstag und Bekanntmachung des Hinweises auf die Patenterteilung:
**20.07.2016 Patentblatt 2016/29**

(21) Anmeldenummer: **12712256.2**

(22) Anmeldetag: **28.03.2012**

(51) Int Cl.:
***C09C 1/00*** (2006.01) ***A61Q 1/02*** (2006.01)
***A61K 8/02*** (2006.01) ***A61K 8/19*** (2006.01)
***C09C 1/40*** (2006.01) ***C09D 5/36*** (2006.01)
***C09D 11/037*** (2014.01) ***C09C 1/28*** (2006.01)

(86) Internationale Anmeldenummer:
**PCT/EP2012/055537**

(87) Internationale Veröffentlichungsnummer:
**WO 2012/130897 (04.10.2012 Gazette 2012/40)**

(54) **WETTERSTABILE PERLGLANZPIGMENTE, VERFAHREN ZU IHRER HERSTELLUNG UND VERWENDUNG**

WEATHERING-RESISTANT PEARL-LUSTRE PIGMENTS, PROCESS FOR PRODUCING SAME AND USE

PIGMENTS NACRÉS STABLES AUX INTEMPÉRIES, PROCÉDÉ DE FABRICATION CORRESPONDANT ET UTILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.03.2011 DE 102011015338**

(43) Veröffentlichungstag der Anmeldung:
**05.02.2014 Patentblatt 2014/06**

(73) Patentinhaber: **Eckart GmbH**
**91235 Hartenstein (DE)**

(72) Erfinder:
- **GRÜNER, Michael**
**91275 Auerbach (DE)**
- **SCHNEIDER, Thomas**
**91207 Lauf a. d. Pegnitz (DE)**
- **KAUPP, Günter**
**91284 Neuhaus (DE)**

(74) Vertreter: **Walcher, Armin**
**Louis, Pöhlau, Lohrentz**
**Patentanwälte**
**Postfach 30 55**
**90014 Nürnberg (DE)**

(56) Entgegenhaltungen:
**WO-A1-97/29059**
**DE-A1- 19 639 783**
**DE-A1-102004 041 586**
**DE-A1-102006 009 129**
**DE-A1-102006 009 130**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 691 478 B1

## Beschreibung

Perlglanzpigmente, die Titandioxid in der Deckschicht enthalten bzw. die aus partikelförmigem $TiO_2$ aufgebaut sind, besitzen eine gewisse photokatalytische Aktivität. Wirkt nun UV-Licht in Gegenwart von Wasser und Sauerstoff auf ein Perlglanzpigment ein, so kann die UV-Aktivität des Perlglanzpigments einen beschleunigten Abbau von organischen Verbindungen, z. B. einer Bindemittelmatrix, auslösen. Bereits der im Tageslicht enthaltene UV-Anteil kann diese Reaktion bedingen, d.h. für Applikationen wie Automobillackierungen, die der Witterung direkt ausgesetzt sind, müssen besonders stabilisierte Perlglanzpigmente eingesetzt werden. Um diesem für die Außenanwendung nachteiligen Effekt entgegenzuwirken, können Perlglanzpigmente zur Verminderung der Photoaktivität mit verschiedenen Schutzbeschichtungen ausgestattet werden. Üblicherweise werden dabei ausgehend von wässrigen Metallsalzlösungen schwerlösliche Verbindungen als Metalloxide auf die Oberfläche der Pigmente gefällt. Hierbei werden vorwiegend zwei unterschiedliche Metalloxide verwendet. Um die Kompatibilität der Pigmente zu unterschiedlichen Lacken, insbesondere aber zu den umweltverträglicheren wasserbasierenden Systemen, zu fördern, wird in der Regel noch eine zusätzliche organische Modifizierung der Deckschicht, z.B. mittels Silanen, aufgebracht.

Gemäß der Lehre der EP 0 632 109 A1 wird eine dreischichtige Schutzschicht auf ein mit Metalloxiden belegtes plättchenförmiges Substrat aufgebracht. In einer 1. Stufe wird eine $SiO_2$ Schicht, in einer 2. Stufe wird ein Hydroxid oder Oxidhydrat von Cer, Aluminium oder Zirkonium und in einer 3. Stufe werden mindestens ein Hydroxid oder Oxidhydrat von Cer, Aluminium oder Zirkonium sowie ein organisches Kupplungsreagenz aufgebracht. Dieser dreischichtige Aufbau ist nachteiligerweise sehr aufwendig und entsprechend kostenintensiv bei der Herstellung. Darüber hinaus müssen die Kupplungsreagenzien vor der Bindung an die Pigmentoberfläche hydrolysiert werden, wobei gemäß der Lehre der EP 0 888 410 B1 jedoch nur maximal 60 % der zugesetzten Kupplungsreagenzien an die Pigmentoberfläche gebunden werden können.

Die EP 0 888 410 B1 offenbart modifizierte Perlglanzpigmente auf der Basis eines mit Metalloxiden beschichteten, plättchenförmigen Substrates. Gemäß der Lehre der EP 0 888 410 B1 besteht die Deckschicht aus mindestens zwei Oxiden, gemischten Oxiden oder Mischoxiden von Siliziumdioxid, Aluminumoxid, Ceroxid, Titandioxid oder Zirkoniumdioxid und einem wasserbasierenden oligomeren Silan. Der Aufbau der Deckschicht ist mithin ebenfalls sehr kompliziert und entsprechend aufwendig in der Herstellung.

Die EP 1 682 622 B1 offenbart ebenfalls eine Deckschicht aus zwei Metalloxiden, wobei hier zwingend zuerst eine Ceroxidschicht und nachfolgend eine $SiO_2$-Schicht gefällt wird. Als Kupplungsreagenzien werden ebenfalls vorwiegend Silane eingesetzt.

Die EP 0 881 998 B1 offenbart wetterstabile Perlglanzpigmente mit einer Deckschicht entweder aus Aluminiumoxid oder einem wiederum zweischichtigen Aufbau aus Aluminiumoxid und Ceroxid sowie Silanen als Kupplungsreagenzien.

Die EP 1 727 864 A1 offenbart wetterstabile Perlglanzpigmente mit einer Deckschicht nur aus $SiO_2$. Diese Pigmente sind jedoch nicht immer in allen Anwendungen völlig wetterstabil, insbesondere bei optisch sehr hochwertigen Pigmenten.

Die 0 141 174 B1 offenbart wetterstabile Perlglanzpigmente mit einer Deckschicht, welche Cerhydroxid enthält. Es wird in dieser Schrift vorgeschlagen, diese Deckschicht durch eine Silikatschicht und bevorzugt durch weitere Oxidschichten wie Aluminiumoxid oder Zinkoxid zu ergänzen, um eine bessere Anbindung von polymeren Siloxanen, die als Kupplungsmittel fungieren können, zu gewährleisten.

Bei dem vorgenannten Stand der Technik wird die UV-Aktivität der hochbrechenden $TiO_2$-Schich meist durch mindestens zwei verschiedene Oxidschichten oder eine Mischschicht zweier Oxide unterdrückt. Die Verwendung verschiedener Oxide beeinträchtigt die optischen Eigenschaften, insbesondere den Glanz der Perlglanzpigmente. Dies kann sich insbesondere nachteilig bemerkbar machen, wenn optisch sehr hochwertige Perlglanzpigmente vorliegen, wie sie heutzutage durch die Verwendung synthetischer Substrate zur Verfügung stehen. Auch sind diese relativ komplexen Schichtsysteme aus mehreren Oxiden oder Mischoxiden aufwendig herzustellen. Für die Fällung jedes Metallhydroxids bzw. Metalloxidhydrats gibt es einen optimalen pH-Bereich. Mischfällungen verschiedener Hydroxide bzw. Oxidhydrate finden daher meist bei pH-Werten statt, die einen Kompromiss der für die Fällung der reinen Hydroxide bzw. der reinen Oxidhydrate optimalen Werte darstellen. Daher sind in der Regel keine Fällungsreaktionen möglich, bei denen die Bedingungen für die Fällung aller beteiligten anorganischer Komponenten jeweils optimal eingestellt sind.

In der EP 1 084 198 B1 sind Effektpigmente beschrieben, die aufgrund ihrer Oberflächenmodifizierung mit reaktiven Orientierungsmitteln, sehr gute Haftfestigkeiten zum Basislack aufweisen. Die EP 1 084 198 B1 offenbart jedoch keine Wetter- und UV-stabilen Perlglanzpigmente.

Die Nachbeschichtung von Perlglanzpigmenten mit Cerhydroxid und $SiO_2$ wird in M. Jäger, U. Schmidt, "Die Barriere macht den Unterschied", Farbe und Lack 8/2007, S. 20 - 25 beschrieben. Perlglanzpigmente mit einer derartigen Nachbeschichtung zeichnen sich im Unterschied zu Perlglanzpigmenten, welche entweder nur eine Cerhydroxid- oder nur eine $SiO_2$-Schicht aufweisen, durch ihre gute Wetterstabilität aus.

DE 10 2006 009 129 A1 betrifft ein wetterstabiles Perlglanzpigment mit zwei Schutzschichten, wobei die erste

Schutzschicht Ceroxid und/oder Ceroxid-Hydrat und/oder Cerhydroxid umfasst und die zweite Schutzschicht im Wesentlichen aus $SiO_2$ besteht. Die zweite Schutzschicht weist weiterhin eine organisch-chemische Nachbeschichtung auf, wobei die organisch-chemische Nachbeschichtung wenigstens ein über wenigstens ein Sauerstoffatom an der zweiten Schutzschicht gebundenes $\alpha$-Silan umfasst.

DE 10 2006 009 130 A1 betrifft wetterstabile Perlglanzpigmente auf Basis von Glasplättchen, welche als Schutzschicht entweder A) eine $SiO_2$ Schicht oder B) eine erste Schicht mit Ceroxid und/oder Ceroxidhydrat und/oder Cerhydroxid und eine zweite Schicht aus $SiO_2$ aufweist. Auf der Schutzschicht wird des Weiteren eine organisch-chemische Oberflächenbeschichtung aufbracht.

DE 10 2004 041 586 A1 betrifft Perlglanzpigmente mit einer ersten Schutzschicht mit Ceroxid und/oder Cerhydroxid und einer zweiten Schutzschicht aus $SiO_2$, auf welche eine organisch-chemische Nachbeschichtung aufgebracht ist.

DE 196 39 783 A1 betrifft modifizierte Perlglanzpigmente für Wasserlacksysteme, wobei die Perlglanzpigmente eine Deckschicht aus mindestens zwei Oxiden und/oder gemischten Oxiden und/oder Mischoxiden aus der Gruppe Siliciumdioxid, Aluminiumoxid, Ceroxid, Titanoxid und Zirkoniumoxid und einem wasserbasierenden oligomeren Silansystem besteht.

Aufgabe der vorliegenden Erfindung ist es, ein wetterstabiles Perlglanzpigment bereit zu stellen, welches die vorgenannten Nachteile aus dem Stand der Technik nicht aufweist. Die wetterstabilen Perlglanzpigmente sollen insbesondere in ihren optischen Eigenschaften nicht durch die Deckschichten beeinträchtigt werden.

Die Aufgabe wurde gelöst durch Bereitstellung eines wetterstabilen Perlglanzpigments, das aus einem mit einem oder mehreren hochbrechenden Metalloxid(en) beschichteten plättchenförmigem Substrat, das aus der Gruppe, die aus synthetischen Glimmerplättchen, Glasplättchen, $SiO_2$-Plättchen, $Al_2O_3$-Plättchen, synthetischen Böhmitplättchen, BiOCl-Plättchen und deren Mischungen besteht, ausgewählt wird und einer Deckschicht besteht, wobei die Deckschicht aus folgenden Schichten besteht:

a) einer Cer-haltigen Schicht, welche aus Ceroxid und/ oder Cerhydroxid und/ oder Ceroxidhydrat besteht und die Cer-haltige Schicht direkt auf der hochbrechenden Metalloxidschicht aufgebracht ist,
b) einer organisch-chemischen Kompatibilisierungsschicht, welche die Reaktionsprodukte aus oligomeren Silanen enthält oder daraus besteht, wobei die oligomeren Silane eine oder mehrere Aminogruppen aufweisen und die organisch-chemische Kompatibilisierungsschicht direkt auf der Cer-haltigen Schicht a) aufgebracht ist.

Eine weitere Aufgabe bestand in der Bereitstellung eines einfachen und kostengünstigen Verfahrens zur Herstellung der erfindungsgemäßen Perlglanzpigmente.

Die Aufgabe wurde gelöst durch ein Verfahren zur Herstellung wetterstabiler Perlglanzpigmente, welches folgende Schritte umfasst:

a) Optional Klassieren des plättchenförmigen Substrates unter Erhalt von Substraten, welche die Kennzahlen $D_{10}$, $D_{50}$, $D_{90}$ aus der Summenhäufigkeitsverteilung der volumengemittelten Größenverteilungsfunktion mit einem Span $\Delta D$ von 0,7 - 1,4 aufweisen,
b) Suspendieren eines plättchenförmigen Substrates, optional aus Schritt a), in wässriger Lösung und Beschichten des plättchenförmigen Substrates mit einem oder mehreren hochbrechenden Metalloxid(en) unter Erhalt von Perlglanzpigmenten,
c) Beschichten der Perlglanzpigmente aus Schritt b) in wässriger Lösung mit einem Cer-salz oder einer hydrolysierbaren Cer-metallorganischen Verbindung unter Erhalt einer Schicht, die aus der Gruppe, die aus Cerhydroxid, Ceroxidhydrat und Mischungen davon besteht, ausgewählt wird,
d) Beschichten der Perlglanzpigmente aus Schritt c) in wässriger Lösung mit oligomeren Silanen,
e) Abtrennen der beschichteten Perlglanzpigmente, optional Waschen mit vollentsalztem Wasser, und Trocknen bei einer Temperatur von bei 80° bis 160°C.

## Substrate

Die vorliegende Erfindung beruht auf wetterstabilen Perlglanzpigmenten, die optisch sehr hochwertige Eigenschaften haben. Hierbei werden insbesondere der Glanz und die Farbreinheit der Perlglanzpigmente im Anwendungsmedium verstanden. Daher basieren die erfindungsgemäßen Perlglanzpigmente ausschließlich auf plättchenförmigen synthetischen Substraten, die im Gegensatz zu beispielsweise natürlichen Glimmerplättchen sehr glatte Oberflächen und scharfe Bruchkanten aufweisen.

Die plättchenförmigen Substrate werden daher der Gruppe bestehend aus synthetischen Glimmerplättchen, Glasplättchen, $SiO_2$-Plättchen, $Al_2O_3$-Plättchen, synthetischen Böhmitplättchen, BiOCl-Plättchen und deren Mischungen, entnommen.

Bevorzugt werden die plättchenförmigen synthetischen Substrate der Gruppe bestehend aus synthetischen Glimmerplättchen, Glasplättchen, $Al_2O_3$-Plättchen und deren Mischungen, entnommen. Besonders bevorzugt sind Glasplättchen und synthetische Glimmerplättchen sowie deren Mischungen.

Derartige synthetische Substrate sind aus einer Reihe von Patentanmeldungen bzw. Patenten bekannt. Besteht z.B. das plättchenförmige synthetische Substrat aus Glasplättchen, so werden im Rahmen dieser Erfindung bevorzugt jene verwendet, die nach den in EP 0 289 240 A1, WO 2004/056716 A1 und der WO 2005/063637 A1 beschriebenen Verfahren hergestellt werden. Die als Substrat verwendbaren Glasplättchen können beispielsweise eine Zusammensetzung entsprechend der Lehre der EP 1 980 594 B1 aufweisen.

Optisch besonders hochwertige Perlglanzpigmente basierend auf synthetischen Substraten sind aus der EP 2 217 664 B1 bekannt, die hiermit unter Bezugnahme aufgenommen ist. Hier werden die Substrate mit einer engen Größenverteilung offenbart, die überraschenderweise die Bereitstellung von Perlglanzpigmenten mit besonders hoher Farbreinheit und hohem Glanz ermöglicht.

Bei einer Ausführungsform weisen die erfindungsgemäßen wetterstabilen Perlglanzpigmente eine Summenhäufigkeitsverteilung der volumengemittelten Größenverteilungsfunktion mit den Kennzahlen $D_{10}$, $D_{50}$ und $D_{90}$ auf, wobei diese Summenhäufigkeitsverteilung einen Span $\Delta D$ in einem Bereich von 0,7 - 1,4 aufweist. Der Span $\Delta D$ wird gemäß Formel (I) berechnet:

$$\Delta D = (D_{90} - D_{10}) / D_{50} \qquad \text{(I)}$$

Zur Charakterisierung der Teilchengrößenverteilung wird erfindungsgemäß der Span $\Delta D$ verwendet. Je kleiner der Span ist, desto enger ist die Teilchengrößenverteilung.

In besonders bevorzugten Ausführungsformen weisen die erfindungsgemäßen wetterstabilen Perlglanzpigmente einen Span $\Delta D$ in einem Bereich von 0,75 -1,3, weiter bevorzugt in einem Bereich von 0,8 bis 1,2 und ganz besonders bevorzugt in einem Bereich von 0,85 bis 1,1 auf.

Oberhalb eines Spans $\Delta D$ von 1,4 werden keine farbreinen Perlglanzpigmente erhalten. Perlglanzpigmente unterhalb eines Spans der Größenverteilung von 0,7 können im Rahmen der üblichen Methoden nur sehr aufwendig und damit nicht mehr wirtschaftlich hergestellt werden.

Die erfindungsgemäßen wetterstabilen Perlglanzpigmente können eine beliebige mittlere Teilchengröße $D_{50}$ aufweisen. Die $D_{50}$-Werte der erfindungsgemäßen Pigmente liegen vorzugsweise in einem Bereich von 3 bis 80 $\mu$m. Bevorzugt weisen die erfindungsgemäßen Pigmente einen $D_{50}$-Wert aus einem Bereich von 5 bis 63 $\mu$m, besonders bevorzugt aus einem Bereich von 7 bis 56 $\mu$m und ganz besonders bevorzugt aus einem Bereich von 9 bis 49 $\mu$m auf.

Die $D_{10}$-Werte der erfindungsgemäßen Pigmente liegen vorzugsweise in einem Bereich von 1 bis 25 $\mu$m. Bevorzugt weisen die erfindungsgemäßen Pigmente einen $D_{10}$-Wert aus einem Bereich von 2 bis 21 $\mu$m, besonders bevorzugt aus einem Bereich von 3 bis 18 $\mu$m und ganz besonders bevorzugt aus einem Bereich von 4 bis 14 $\mu$m auf.

Die $D_{90}$-Werte der erfindungsgemäßen Pigmente liegen vorzugsweise in einem Bereich von 6 bis 250 $\mu$m. Bevorzugt weisen die erfindungsgemäßen Pigmente einen $D_{90}$-Wert aus einem Bereich von 15 bis 210 $\mu$m auf.

Der $D_{10}$-, $D_{50}$- bzw. $D_{90}$-Wert der Summenhäufigkeitsverteilung der volumengemittelten Größenverteilungsfunktion, wie sie durch Laserbeugungsmethoden erhalten wird, gibt an, dass 10%, 50% bzw. 90% der erfindungsgemäßen Pigmente einen Durchmesser aufweisen, der gleich oder kleiner als der jeweils angegebene Wert ist. Hierbei wird die Größenverteilungskurve der Pigmente mit einem Gerät der Fa. Malvern (Gerät: MALVERN Mastersizer 2000) gemäß Herstellerangaben bestimmt. Die Auswertung der Streulichtsignale erfolgte dabei nach der Fraunhofer Methode.

Die mittlere Dicke der zu beschichtenden plättchenförmigen synthetischen Substrate liegt vorzugsweise in einem Bereich von 50 nm bis 5000 nm, bevorzugt in einem Bereich von 60 nm bis 3000 nm und besonders bevorzugt in einem Bereich von 70 nm bis 2000 nm.

Bei einer Ausführungsform liegt die mittlere Dicke für Glasplättchen als zu beschichtendes Substrat vorzugsweise in einem Bereich von 750 nm bis 1500 nm. Derartige Glasplättchen sind kommerziell auf breiter Basis verfügbar. Weitere Vorteile bieten dünnere Glasplättchen. Dünnere Substrate führen zu einer geringeren Gesamtschichtdicke der erfindungsgemäßen Pigmente. So sind ebenfalls bevorzugt Glasplättchen, deren mittlere Dicke in einem Bereich von 100 nm bis 700 nm, weiter bevorzugt in einem Bereich von 150 nm bis 600 nm, besonders bevorzugt in einem Bereich von 170 nm bis 500 nm und ganz besonders bevorzugt in einem Bereich von 200 nm bis 400 nm liegt.

Bei einer weiteren Ausführungsform liegt die mittlere Dicke für synthetischen Glimmer als zu beschichtendes plättchenförmiges Substrat bevorzugt in einem Bereich von 100 nm bis 700 nm, weiter bevorzugt in einem Bereich von 120 nm bis 600 nm, besonders bevorzugt in einem Bereich von 140 nm bis 500 nm und ganz besonders bevorzugt in einem Bereich von 150 nm bis 450 nm.

Beschichtet man plättchenförmige synthetische Substrate unterhalb einer mittleren Dicke von 50 nm mit beispielsweise hochbrechenden Metalloxiden, so werden extrem bruchempfindliche Pigmente erhalten, die schon beim

Einarbeiten in das Anwendungsmedium zerbrechen können, was wiederum eine signifikante Herabsetzung des Glanzes bedingt. Zudem dauern die Beschichtungszeiten dieser dünnen Substrate mit beispielsweise hochbrechenden Metalloxiden aufgrund der hohen spezifischen Oberflächen (Oberfläche pro Gewichtseinheit Pigment) dieser nichtmetallischen plättchenförmigen synthetischen Substrate sehr lange, was hohe Herstellkosten verursacht. Oberhalb einer mittleren Substratdicke von 5000 nm können die Pigmente insgesamt zu dick werden. Damit kann eine schlechtere spezifische Deckfähigkeit, d.h. abgedeckte Fläche pro Gewichtseinheit an erfindungsgemäßem Pigment, sowie eine geringere planparallele Orientierung im Anwendungsmedium verbunden sein. Aus einer schlechteren Orientierung wiederum resultiert ein verminderter Glanz.

Bei einer bevorzugten Ausführungsform beträgt die Standardabweichung der Dicke der künstlichen Substrate 15 % bis 100 % und besonders bevorzugt 20 bis 70 %. Unterhalb einer Standardabweichung von 15 % werden farbflopende Effektpigmente erhalten. Oberhalb einer Standardabweichung von 100 % sind derart viele dickere Pigmente in dem gesamten Pigmentensemble enthalten, dass es dann zu schlechterer Orientierung und damit zu Glanzverlusten kommt.

Die mittlere Dicke wird anhand eines gehärteten Lackfilmes, in dem die Effektpigmente im Wesentlichen planparallel zum Untergrund ausgerichtet sind, bestimmt. Hierzu wird ein Querschliff des gehärteten Lackfilmes unter einem Rasterelektronenmikroskop (REM) untersucht, wobei die Dicke von mindestens 100 Perlglanzpigmenten bestimmt und statistisch gemittelt wird.

Die Substrate werden mit wenigstens einer hochbrechenden Metalloxidschicht beschichtet, um den üblichen, auf Interferenz basierenden, Perlglanzeffekt zu erhalten. Im Rahmen dieser Erfindung wird unter einer hochbrechenden Metalloxidschicht eine Schicht mit einem Brechungsindex > 1,8, bevorzugt > 2,0 verstanden.

Vorzugsweise enthält oder besteht die wenigstens eine hochbrechende Schicht aus Metalloxiden, Metallhydroxiden und/oder Metalloxidyhdraten aus der Gruppe, bestehend aus $TiO_2$, $Fe_2O_3$, $Fe_3O_4$, $TiFe_2O_5$, $Fe_2Ti_3O_9$, $FeTiO_3$, ZnO, $SnO_2$, CoO, $Co_3O_4$, $ZrO_2$, $Cr_2O_3$ $VO_2$, $V_2O_3$, $(Sn, Sb)O_2$ und deren Mischungen. Besonders bevorzugt enthält oder besteht die wenigstens eine hochbrechende Schicht aus Metalloxiden, Metallhydroxiden und/oder Metalloxidyhdraten aus der Gruppe, bestehend aus $TiO_2$, $Fe_2O_3$, $Fe_3O_4$, $TiFe_2O_5$, $Fe_2Ti_3O_9$, $FeTiO_3$ und deren Mischungen.

Bei einer weiter bevorzugten Ausführungsform ist das Substrat mit nur einer (Zahl: 1) hochbrechenden Metalloxidschicht bestehend aus der Gruppe $TiO_2$, $Fe_2O_3$, $TiFe_2O_5$, $Fe_2Ti_3O_9$, $FeTiO_3$ und deren Mischungen, beschichtet.

## Cer-haltige Schicht

Die Cer-haltige Schicht besteht aus Ceroxid und/ oder Cerhydroxid und/ oder Ceroxidhydrat. Ceroxid entsteht nach Trocknen der Pigmente bei erhöhter Temperatur.
Es hat sich überraschenderweise gezeigt, dass die Cer-haltige Schicht schon in sehr geringen Mengen bereits einen ausreichenden UV-Schutz ermöglicht, obwohl in der vorliegenden Erfindung auf eine zweite Schutzschicht verzichtet wird.

In bevorzugten Ausführungsformen liegt die Menge der Cer-haltigen Schicht in einem Bereich von 0,05 bis unter 0,9 Gew.-%, bezogen auf das Gesamtgewicht des Perlglanzpigments. Dabei wird die Menge der Cer-haltigen Schicht auf elementares Cer umgerechnet.

Der Gewichtsanteil sollte, bezogen auf die eingesetzte Menge an Perlglanzpigment, vorzugsweise nicht über 0,9 Gew.-% liegen, da andernfalls Verluste der optischen Qualität des Pigmentes zu stark sein könnten. Außerdem macht sich bei Mengen über 0,9 Gew.-% ein störender Gelbstich bemerkbar. Dieser Gelbstich ist bei der vorliegenden Erfindung deshalb so auffallend, weil die erfindungsgemäßen Perlglanzpigmente optisch sehr hochwertige Eigenschaften aufweisen und schon kleine Beeinträchtigungen sehr störend wirken. Unterhalb eines Gewichtsanteils von 0,05 Gew.-% wiederum ist die zusätzliche UV-Stabilisierung in der Regel nicht ausgeprägt genug. Im Einzelfall hängt der Gewichtsanteil von der Feinheit und damit einhergehend von der spezifischen Oberfläche des Perlglanzpigmentes sowie von der Schichtdicke der $TiO_2$-Schicht ab. Feinere Pigmente und dickere $TiO_2$-Schichten benötigen im Allgemeinen auch einen höheren Gehalt an Ceroxid und/oder Cerhydroxid und/oder Ceroxidhydrat.

In besonders bevorzugten Ausführungsformen liegt die Menge der Cer-haltigen Schicht in einem Bereich von 0,1 bis 0,7 Gew.-%, weiter bevorzugt in einem Bereich von 0,2 bis 0,5 Gew.-%, jeweils bezogen auf das Gesamtgewicht des Perlglanzpigments.

Der Ceroxidgehalt der Cer-haltigen Schicht wird beispielsweise mittels Röntgenfluoreszenzanalyse (RFA) bestimmt. Hierzu werden die Pigmente mit Lithiumtetraborat versetzt, in oxidierender Atmosphäre geschmolzen und als homogene Glastablette vermessen. Als Messgerät diente das Gerät Advantix ARL, Fa. Thermo Scientific. Eine weitere Methode zur Bestimmung des Ceroxidgehalts ist XPS. Bei dieser Methode kann durch Sputtern zusätzlich ein Tiefenprofil der Elemente der Oberflächenbeschichtung erhalten werden. Bevorzugt wird der Ceroxidgehalt der Cer-haltigen Schicht mittels Röntgenfluoreszenzanalyse ermittelt.

In äußerst bevorzugten Ausführungsformen erfolgt die Beschichtung der Perlglanzpigmente mit der Cer-haltigen Schutzschicht in wässrigem Milieu. Es wird vermutet, dass sich hierbei - im Gegensatz zu der in der EP 1 682 622

B1 beschriebenen Route der Abscheidung aus organischen Lösemitteln - eine besser strukturierte, gleichmäßigere Schutzschicht ausbildet. Daher scheint man auch nur sehr wenig Material zu benötigen, um eine ausreichende UV- und Wetterstabilität zu erhalten.

**Wasserbasierende, oligomere Silane**

Das wasserbasierende, oligomere Silan ist aus EP 0 675 128, EP 0 716 127 und EP 0 716 128 bekannt. Eine Verwendung als organische Kompatibilisierungsschicht bei wetterstabilen Perlglanzpigmenten ist aus der 0 888 410 B1 bekannt. Diese wasserbasierenden, oligomeren Silane enthalten mindestens eine Art von funktionellen Gruppen. Unter einer funktionellen Bindungsgruppe ist im Rahmen dieser Erfindung eine funktionelle Gruppe zu verstehen, die eine chemische Wechselwirkung zum Bindemittel einzugehen vermag. Die chemische Wechselwirkung kann dabei aus einer kovalenten Bindung, einer Wasserstoffbrückenbindung oder einer ionischen Wechselwirkung bestehen.
Die Wahl einer geeigneten funktionellen Gruppe hängt von der chemischen Natur des Bindemittels ab. Bevorzugt wählt man eine den Funktionalitäten des Bindemittels chemisch kompatible funktionelle Gruppe, um eine gute Anbindung zu ermöglichen. Diese Eigenschaft ist im Hinblick auf wetterstabile und UV-stabile Perlglanzpigmente sehr wichtig, da auf diese Weise eine genügend große Haftfestigkeit zwischen Pigment und ausgehärtetem Bindemittel gegeben ist. Dies ist beispielsweise in Haftprüfungstests wie dem Gitterschnitttest bei Schwitzwassertestbelastungen gemäß der DIN 50 017 nachzuprüfen. Das Bestehen eines derartigen Testes stellt eine notwendige Bedingung für die Verwendung von wetterstabilen Perlglanzpigmenten im Automobillack dar.
Diese wasserbasierenden, oligomeren Silane enthalten zwingend Aminogruppen als funktionelle Gruppe. Die Aminofunktion ist eine funktionelle Gruppe, die mit den meisten in Bindemitteln vorhandenen Gruppen eine oder mehrere chemische Wechselwirkungen eingehen kann. Dies kann eine kovalente Bindung, wie z.B. mit Isocyanat- oder Carboxylatfunktionen des Bindemittels, oder Wasserstoffbrückenbindungen wie mit OH- oder COOR- Funktionen oder auch ionische Wechselwirkungen beinhalten. Eine Aminofunktion ist daher für den Zweck der chemischen Anbindung des Perlglanzpigmentes an verschiedenartige Bindemittel sehr gut geeignet.
In weiter bevorzugten Ausführungsformen weisen die wasserbasierenden, oligomeren Silane Alkylgruppen von 1 bis 18 C-Atomen auf. Durch die Alkylgruppen wird die Pigmentoberfläche teilweise hydrophobiert, was eine Abstoßung von Wasser und eine bessere planparallele Orientierung im Anwendungsmedium ermöglicht. Weiter bevorzugt enthalten die wasserbasierenden, oligomeren Silane Alkylgruppen von 2 bis 10 C-Atomen und besonders bevorzugt von 3 bis 6 C-Atomen.
Die Alkylgruppen können dabei linear, verzweigt und gegebenenfalls ringförmig sein.
Die wasserbasierenden, oligomeren Silane werden bevorzugt durch

- Mischen wasserlöslicher Aminoalkylalkoxysilane der allgemeinen Formel II

  $$R\text{-}Si\text{-}(R^1)_y(OR^{1*})_{3-y}, \qquad (II)$$

  vorzugsweise Aminopropyltriethoxysilan, Aminopropylmethyldiethoxysilan, Aminopropyltrimethoxysilan oder Aminopropylmethyldimethoxysilan, mit nicht wasserlöslichen Alkyltrialkoxysilanen der allgemeinen Formel IIIa

  $$R^2\text{-}Si(OR^{1**})_3 \qquad (IIIa)$$

  vorzugsweise Propyltrimethoxysilan, Propyltriethoxysilan, Methyltriethoxysilan, Methyltrimethoxysilan, Ethyltrimethoxysilan, Ethyltriethoxysilan, Isobutyltrimethoxysilan oder Isobutyltriethoxysilan, und/oder nicht wasserlöslichen Dialkyldialkoxysilanen der allgemeinen Formel IV

  $$AA'\text{-}Si(OR^{1***})_2 \qquad (IV)$$

  vorzugsweise Dimethyldimethoxysilan, Dimethyldiethoxysilan, Methylpropyldimethoxysilan oder Methylpropyldiethoxysilan, und/oder Mischungen aus nicht wasserlöslichen Alkyltrialkoxysilanen und Dialkyldialkoxysilanen der allgemeinen Formeln III und IV,

  wobei R eine aminofunktionelle organische Gruppe ist,
  $R^1$, $R^{1*}$, $R^{1**}$ und $R^{1***}$ einen Methyl-oder Ethyl-Rest,
  $R^2$ einen linearen oder cyclischen oder verzweigten Alkyl-Rest mit 1 bis 8 C-Atomen,
  A einen unverzweigten oder verzweigten Alkyl-Rest mit 1 bis 3 C-Atomen und
  A' einen unverzweigten oder verzweigten Alkyl-Rest mit 1 bis 3 C-Atomen darstellen und $0 < y \leq 1$ ist,

- Versetzen des Gemisches mit Wasser und
- Einstellen des pH-Wertes der Reaktionsmischung auf einen Wert zwischen 1 und 8 und
- Entfernen des bereits vorhandenen und/oder bei der Umsetzung entstandenen Alkohols hergestellt.

Das oligomere Silan ist weiterhin herstellbar durch

- Mischen von Q Molen wasserlöslicher Aminoalkylalkoxysilane der allgemeinen Formel II $R\text{-}Si(R^1)_y(OR^{1*})_{3-y}$ vorzugsweise Aminopropyltriethoxysilan, Aminopropylmethyldiethoxysilan, Aminopropyltrimethoxysilan oder Aminopropylmethyldimethoxysilan, mit M Molen nicht wasserlöslicher Alkylalkoxysilane der allgemeinen Formel IIIb

$$R^3\text{-}Si(OR^{1**})_3 \qquad \text{(IIIb)}$$

wobei R eine aminofunktionelle organische Gruppe ist,
$R^1$, $R^{1*}$ und $R^{1**}$ einen Methyl-oder Ethyl-Rest und
$R^3$ einen linearen oder cyclischen oder verzweigten Alkylrest mit 1 bis 6 C-Atomen oder eine Ureido-Alkylgruppe der allgemeinen Formel V

$$NH_2\text{-}CO\text{-}NH\text{-}(CH_2)_b\text{-}, \text{ mit } 1 < b < 6 \qquad \text{(V)}$$

vorzugsweise b = 3, darstellt und $0 < y \leq 1$ ist,
in dem molaren Verhältnis $0 < M/Q \leq 2$,

- Versetzen des Gemisches mit Wasser,
- Einstellen des pH-Wertes der Reaktionsmischung auf einen Wert zwischen 1 und 8 und
- optional Entfernen des bereits vorhandenen und/ oder bei der Umsetzung entstandenen Alkohols.

Ferner ist das oligomere Silan erhältlich durch

- Mischen wasserlöslicher Organosilane der allgemeinen Formel VI

$$H_2N(CH_2)_f(NH)_g(CH_2)_i\text{-}Si(CH_3)_h(OR^0)_{3-h} \qquad \text{(VI)}$$

worin $0 \leq f \leq 6$, g = 0 falls f = 0, g = 1 falls f > 1,$0 \leq i \leq 6$.$0 \leq h \leq 1$ und $R^0$ eine Methyl-, Ethyl-, Propyl-oder Isopropyl-Gruppe sind, vorzugsweise Aminopropyltriethoxysilan, mit wasserlöslichen, jedoch in wässrigem Medium nicht stabilen Organosilanen der allgemeinen Formel VII

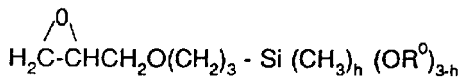

(VII)

worin $0 < h \leq 1$ ist und $R^0$ einen Methyl-, Ethyl-, Propyl- oder Isopropyl-Rest darstellt, vorzugsweise Glycidyloxypropyltrimethoxysilan, und/ oder der allgemeinen Formel VIII

$$H_2C{=}CR'\text{-}COO(CH_2)_3\text{-}Si(CH_3)_h(OR^0)_{3-h} \qquad \text{(VIII)}$$

worin $0 \leq h \leq 1$ ist, $R^0$ einen Methyl-, Ethyl-, Propyl-oder Isopropyl-Rest und R' einen Methyl-oder Wasserstoff-Rest darstellen, vorzugsweise Methacryloxypropyltrimethoxysilan, und ein nicht wasserlösliches Organosilan der allgemeinen Formel IX

$$R''\text{-}Si(CH_3)_h(OR^0)_{3-h} \qquad \text{(IX)}$$

worin $0 \leq h \leq 1$ ist, $R^0$ einen Methyl-, Ethyl-, Propyl- oder Isopropyl-Rest und R" einen linearen, verzweigten oder cyclischen Kohlenwasserstoff-Rest mit 1 bis 8 C-Atomen darstellen, vorzugsweise Propyltrimethoxysilan,
in dem molaren Verhältnis M = a / (b + c + d), wobei a die Summe der Molzahlen der Organosilane gemäß Formel VI, b die Summe der Molzahlen der Organosilane gemäß Formel VII sowie c die Summe der Molzahlen der Organosilane gemäß Formel VIII und d die Summe der Molzahlen der Organosilane gemäß Formel IX sind, mit $0 \leq M \leq$

3, insbesondere für M gleich 0 mit a gleich 0 und/oder c gleich d gleich 0 und b ≥ 1 sowie vorzugsweise 0,5 ≤ M ≤ 3,

- Versetzen des Gemisches mit einem Wasser/Säure-Gemisch,
- Einstellen des pH-Wertes der Reaktionsmischung auf einen Wert zwischen 1 und 8 und
- optional Entfernen des bereits vorhandenen und/oder bei der Umsetzung entstandenen Alkohols.

Bevorzugt gibt man während der destillativen Abtrennung des Alkohols Wasser in dem Masse zu, wie Alkohol bzw. Alkohol/ Wasser-Gemisch aus dem Reaktionsmedium entfernt wird. Zum Einstellen des pH-Wertes sind einbasige Säuren besonders geeignet. So hergestellte Produkte setzen auch bei Verdünnen mit Wasser keine weiteren Alkohole durch Hydrolyse frei und weisen einen Flammpunkt von deutlich mehr als 70°C auf.

Der Anteil der Deckschicht am gesamten Perlglanzpigment liegt bevorzugt in einem Bereich von 0,3 bis 2,5 Gew.-%, besonders bevorzugt in einem Bereich von 0,4 bis 2,1 Gew.-% und ganz besonders bevorzugt in einem Bereich von 0,5 bis 1,8 Gew.-%, jeweils bezogen auf das Gesamtgewicht des Perlglanzpigments. Dieser überraschend niedrige Anteil der gesamten Deckschicht im erfindungsgemäßen Perlglanzpigment ist auf die besonders vorteilhafte Kombination der beiden Komponenten zurückzuführen.

Die wasserbasierenden oligomeren Silane binden offensichtlich hervorragend an die Cer-haltige Schicht an und daher wird im Gegensatz zum Stand der Technik keine weitere Metalloxidschicht in der Deckschicht benötigt. Durch den geringen Ceroxidgehalt werden die optischen Eigenschaften des erfindungsgemäßen Perlglanzpigments nicht beeinträchtigt. Mithilfe der wasserbasierenden oligomeren Silansysteme lassen sich auch in Wasser nicht oder schwer lösliche Gruppen, wie beispielsweise Alkylgruppen von Silanen, problemlos auf die Oberfläche der Perlglanzpigmente anbinden.

Die erhaltenen wasserbasierenden oligomeren Silane (organopolysiloxan-haltige Zusammensetzungen auf Wasserbasis) sind im Wesentlichen frei von organischen Lösemitteln und besitzen einen Flammpunkt von mehr als 70°C. Da durch die Mischung mit Wasser die Alkoxygruppen bereits im Wesentlichen hydrolysiert wurden, werden beim Verdünnen mit Wasser weniger als 5 Gew.-% Alkohole (Methanol, Ethanol) durch Hydrolyse freigesetzt.
Es resultieren beispielsweise Verbindungen mit folgender Näherungsstruktur (Formel X)

$$[HO[Si(A)(OH)_Z(CH_3)_{1-Z}O]_a[Si(B)(OH)_Y(CH_3)_{1-Y}O]_b[Si(C)(OH)_W(CH_3)_{1-W}O]_c[Si(D)(OH)_V(CH_3)_{1-V}O]_d[H\cdot(HX)]_e \quad (X)$$

wobei

A = Aminoalkylrest abgeleitet aus der allgemeinen Formel VI,
B = Glycidetheralkylrest abgeleitet der allgemeinen Formel VII,
C = Acryloxyalkyl-oder Methacryloxyalkylrest der allgemeinen Formel VIII,
D = Alkylrest der allgemeinen Formel IX,
HX = einbasige Säure, wobei X = anorganischer oder organischer Säurerest, wie z.B. Chlorid, Nitrat, Formiat, Acetat, v gleich 0 oder 1 und w gleich 0 oder 1 und y gleich 0 oder 1 und z gleich 0 oder 1 und a + b + c + d ≥ 4 und a ≤ e ≤ 2a sind, mit 0 ≤ a / (b + c + d) ≤ 3, insbesondere für a / (b + c + d) gleich 0 mit a = 0 und/oder c gleich d gleich 0 und b ≥ 1 sowie für 0,5 ≤ a / (b + c + d) ≤ 3.

Aufgabe der siliziumfunktionellen Hydroxylgruppen ist es, chemische Bindungen zu den Hydroxylgruppen der Cer-haltigen Schicht auf der Perlglanzpigmentoberfläche auszubilden. Dadurch wird ein stabiler Verbund zwischen Silan und Pigmentoberfläche aufgebaut.
Die organofunktionellen Gruppen des oligomeren Silans haben die Aufgabe, Bindungen zum Polymer des Wasserlackes herzustellen. Da die Oligomeren mit mehreren, sich voneinander unterscheidenden funktionellen Gruppen ausgerüstet werden können, kann das Pigment in unterschiedlichen Wasserlacksystemen verwendet werden. Eine Ausrüstung mit Methacryl- und Aminogruppen bedeutet zum Beispiel, dass das Pigment für Wasserlacksysteme mit Polyester als Polymer und für Wasserlacksysteme mit Polyurethan als Polymer einsetzbar ist.

Die erhaltenen wasserbasierenden oligomeren Silane (organopolysiloxan-haltige Zusammensetzungen auf Wasserbasis) sind vorteilhaft im Wesentlichen frei von organischen Lösemitteln und besitzen einen Flammpunkt von mehr als 70°C. Da durch die Mischung mit Wasser die Alkoxygruppen bereits im Wesentlichen hydrolysiert wurden, werden beim Verdünnen mit Wasser weniger als 5 Gew.-% Alkohole, wie z.B. Methanol oder Ethanol, durch Hydrolyse freigesetzt.

Beispiele der wasserbasierenden oligomeren Silane sind wässriges, alkoholfreies Aminosilanhydrolysat (Dynasylan Hydrosil 1151), wässriges, alkoholfreies amino/alkylfunktionelles Siloxancooligomer (Dynasylan Hydrosil 2627), wässriges, alkoholfreies diamino/ alkylfunktionelles Siloxancooligomer (Dynasylan Hydrosil 2776), wässriges, alkoholfreies amino/ vinylfunktionelles Siloxancooligomer (Dynasylan Hydrosil 2907), wässriges, alkoholfreies amino/alkylfunktionelles Siloxancooligomer (Dynasylan Hydrosil 2909), wässriges, alkoholfreies amino/alkylfunktionelles Siloxancooligomer (Dynasylan Hydrosil 2909), Hydrosil 2926) oder wässriges, alkoholfreies amino/methacrylatfunktionelles Siloxancooligomer (Dynasylan Hydrosil 2929), oligomeres Diaminosilansystem (Dynasylan 1146).

Zusätzlich können weitere Silane Verwendung finden.

Es ist äußerst bevorzugt, dass die Beschichtung der Perlglanzpigmente mit der gesamten Deckschicht in wässrigem Milieu erfolgt.

Eine Beschichtung im wässrigen Milieu ist preisgünstiger als jene in organischem Medium und vermeidet das Problem der Entsorgung organischer Lösemittel.

Weiterhin wird die Aufgabe gemäß der vorliegenden Erfindung durch ein Verfahren zur Herstellung wetterstabiler Perlglanzpigmente gelöst, wobei dieses folgende Schritte umfasst:

a) Optional Klassieren der plättchenförmigen Substrate unter Erhalt von Substraten , welche die Kennzahlen $D_{10}$, $D_{50}$, $D_{90}$ aus der Summenhäufigkeitsverteilung der volumengemittelten Größenverteilungsfunktion mit einem Span $\Delta D$ von 0,7 - 1,4 aufweisen,
b) Suspendieren der plättchenförmigen Substrate aus Schritt a) in wässriger Lösung,
c) Beschichten der Perlglanzpigmente aus Schritt b) in wässriger Lösung mit einem Cer-salz oder einer hydrolisierbaren Cer-metallorganischen Verbindung unter Erhalt einer Schicht entnommen der Gruppe bestehend aus Cerhydroxid, Ceroxidhydrat und deren Mischungen,
d) Beschichten der Perlglanzpigmente aus Schritt c) in wässriger Lösung mit wasserbasierenden, oligomeren Silanen,
e) Abtrennen der beschichteten Perlglanzpigmente, gegebenenfalls Waschen mit vollentsaztem Wasser und Trocknen bei einer Temperatur von bei 80° bis 160°C.

Zur Fällung der Cer-haltigen Schicht werden bevorzugte Salze oder hydrolysierbare Cer-metallorganischen Verbindungen wie Cer(III)acetat, Cer(III)acetylacetonat, Cer(III)nitrat, Cer(III)chlorid, Cer(III) sulfat oder Cer(IV)ammoniumnitrat verwendet. Die Fällung wird bei einer Temperatur von 30 bis 100°C, vorzugsweise 40 bis 75°C, durchgeführt. Der pH-Wert dieser Fällung beträgt vorzugsweise von 3 bis 7 und wird, wenn nötig, durch gleichzeitige Zugabe von Säure oder Lauge oder einem geeignetem Puffersystem konstant gehalten.
Die Fällung des wasserbasierenden oligomeren Silans erfolgt bei einer Temperatur aus einem Bereich von 75 bis 80°C und einem pH-Wert aus einem Bereich von 5 bis 11.

Der Einfluss der Beschichtung auf die optischen Eigenschaften von Perlglanzpigmenten wird insbesondere nach Schwitzwasserbelastung deutlich. In einem Basislack-/Klarlacksystem erlaubt der Schwitzwassertest außerdem eine Aussage zur Benetzung und Einbettung der Perlglanzpigmente in die Bindemittelmatrix. Nach Belastung unter exakt definierten Bedingungen im Schwitzwassertest gemäß DIN 50 017 (Kondenswasser-Konstantklimate) werden zum einen die Haftung mittels Gitterschnittest als auch die optischen Eigenschaften, wie Abbildungsschärfe (DOI: distinctness of image), Quellverhalten oder Glanz, im Vergleich zu einer unbelasteten Probe beurteilt. Trotz Schwitzwasserbelastung werden die optischen Eigenschaften der erfindungsgemäßen Perlglanzpigmente nur unwesentlich beeinträchtigt. Auch im Gitterschnittest zeigen die erfindungsgemäßen Perlglanzpigmente überraschend gute Ergebnisse. Es hat sich gezeigt, dass der Einsatz eines oligomeren Silansystems auf der beschichteten Pigmentoberfläche ein Eindringen von Wasser oder Feuchtigkeit in die Beschichtung deutlich erschwert. Es wird angenommen, dass dies auf eine verbesserte Kombination von Silan-Vernetzungsgrad und Silan-Anbindungsgrad an die beschichtete Pigmentoberfläche zurückzuführen ist. Bei einem erfindungsgemäß einzusetzenden oligomeren Silansystem ist bereits aufgrund der Oligomerisierung ein erhöhter Silan-Vernetzungsgrad gegeben. Hierdurch wird eine homogene Oberflächenmodifizierung auf der beschichteten Pigmentoberfläche gewährleistet. Der Silan-Anbindungsgrad definiert die Bindungsstärke zwischen der beschichteten Pigmentoberfläche und dem erfindungsgemäß einzusetzenden oligomeren Silansystem.
Ein Vergleich der erfindungsgemäßen Perlglanzpigmente mit Perlglanzpigmenten, welche nur eine Cer-haltige Schicht aufweisen zeigt deutlich, dass letztere im Schwitzwassertest sowohl einen Verlust ihrer optischen Eigenschaften als auch im Gitterschnittest nicht die gewünschte Haftfestigkeit aufweisen. Überraschenderweise zeigen die erfindungsgemäßen Perlganzpigmente im Schwitzwassertest vergleichbare oder sogar bessere Ergebnisse als Perlglanzpigmente, welche sowohl eine Cer-haltige als auch eine $SiO_2$-Schicht, welche mit monomeren Silanen oberflächenmodifiziert wurde, im Schichtaufbau aufweisen. Dass der Ersatz einer mit monomeren Silanen oberflächenmodifizierten $SiO_2$-Schicht durch ein oligomeres Silansystem zu keinem Verlust der Haftungs- und optischen Eigenschaften unter den Bedingungen des Schwitzwassertests führt, war nicht vorhersehbar. Im Hinblick auf aus dem Stand der Technik bekannte Pigmente, welche eine $SiO_2$-Schicht als zusätzliche Barriereschicht aufweisen, war es überraschend, dass auch ohne diese zusätzliche Barriereschicht wetterstabile Pigmente erhalten werden können. Neben einer Kosteneinsparung im Herstellungsprozess ist es äußerst vorteilhaft, dass durch Einsparung einer Schicht Perlglanzpigmente erhalten werden können, die die Anforderungen, wie z.B. Wetterstabilität erfüllen, und trotzdem die optischen Eigenschaften durch zusätzliche Schichten nur unwesentlich beeinflusst werden.

Oligomere Silansysteme besitzen gegenüber monomeren Silanen bzw. Mischungen aus monomeren Silanen

den Vorteil, dass bei Verwendung der oligomeren Silansysteme eine vorvernetzte Zusammensetzung auf der beschichteten Pigmentoberfläche aufgebracht wird. Bei Verwendung monomerer Silane wird jedoch aufgrund der Konkurrenzsituation zwischen unterschiedlich reaktiven Silankomponenten und OH-Gruppen der beschichteten Pigmentoberflächen eine Beschichtung mit variablem Vernetzungsgrad aufgebracht. Beispielsweise kann nachgewiesen werden, dass der Silan-Vernetzungsgrad hochreaktiver monomerer Aminosilane wie Dynasylan AMEO auf der beschichteten Pigmentoberfläche sehr unterschiedlich und daher die Oberflächenmodifizierung inhomogen sein kann. Auch der Silan-Anbindungsgrad an die beschichtete Pigmentoberfläche kann aufgrund der Konkurrenzsituation zwischen den OH-Gruppen der monomeren Silane und den reaktiven OH-Gruppen auf der Oberfläche der zu beschichtenden Pigmente variieren und somit die Inhomogenität der Oberflächenmodifizierung noch verstärken. Diese Inhomogenität der Oberflächenmodifierung wird insbesondere unter den Bedingungen des Schwitzwassertests in verschlechterten optischen und Haftungseigenschaften deutlich.

Ein weiterer, insbesondere für Außenanwendungen und in Automobillackierungen nicht zu vernachlässigender Faktor ist die Witterungsstabilität der eingesetzten Pigmente. Die erfindungsgemäßen Perlglanzpigmente zeichnen sich in einer beschleunigten Bewitterungsprüfung durch ihre geringen Farbtonabweichungen bzw. ihren geringen Glanzverlust aus.

Aufgrund der UV-Aktivität titandioxidhaltiger Perlglanzpigments, die einen beschleunigten Abbau von organischen Verbindungen, z. B. einer Bindemittelmatrix, auslösen kann, werden in Außenanwendungen stabilisierte Perglanzpigmente eingesetzt. Um die Wirksamkeit der Stabilisierung zu überprüfen, werden Lackapplikationen von Perlglanzpigmenten UV-Licht ausgesetzt und anschließend farbmetrisch im Vergleich zu den entsprechenden unbelasteten Proben vermessen. Die Farbtonabweichung $\Delta E^*$ ist ein Maß für die Lichtechtheit des pigmentierten Lacks. Die erfindungsgemäßen Perlglanzpigmente beeinflussen die optischen Eigenschaften eines melaminhaltigen Lackes nach UV-Belastung nur unwesentlich.

Gegenstand der Erfindung ist außerdem die Verwendung der erfindungsgemäßen Perlglanzpigmente zur Pigmentierung von Lacken, Druckfarben, Kunststoffen und Kosmetika. Hierfür können sie als Mischungen mit handelsüblichen Pigmenten, beispielsweise anorganischen und organischen Absorptionspigmenten, Effektpigmenten, wie Metalleffektpigmenten und Perlglanzpigmenten und/ oder LCP-Pigmenten, eingesetzt werden.

Bevorzugte Verwendungen der erfindungsgemäßen wetterstabilen Perlglanzpigmente sind Beschichtungen, Lacke, Automobillacke, Pulverlacke und Druckfarben.

Bei einer weiteren Ausführungsform umfasst die vorliegende Erfindung wetterstabile Perlglanzpigmente basierend auf synthetischen Glimmerplättchen, welche mit wenigstens einer hochbrechenden Metalloxidschicht sowie einer Deckschicht aus a) einer Cer-haltigen Schicht und b) einer organischen Kompatibilisierungsschicht belegt ist und Anteil der Deckschicht am gesamten Perlglanzpigment 0,3 bis 2,5 Gew.-%, bezogen auf das Gesamtgewicht des Perlglanzpigments beträgt.

Bei einer weiteren Ausführungsform umfasst die Erfindung wetterstabile Perlglanzpigmente basierend auf Glasplättchen, welche mit wenigstens einer hochbrechenden Metalloxidschicht sowie einer Deckschicht aus a) einer Cer-haltigen Schicht und b) einer organischen Kompatibilisierungsschicht belegt ist, wobei die organische Kompatibilisierungsschicht aus wasserbasierenden oligomeren Silanen besteht, und die Silane Aminogruppen umfassen.

## Beispiele

Die folgenden Beispiele sollen die Erfindung näher erläutern, ohne sie jedoch zu beschränken. Alle Prozentangaben sind als Gew.-% zu verstehen.

## I Herstellung der Pigmente

### Beispiel 1

150 g kommerziell erhältliches silbernes Perlglanzpigment auf Basis von Glasplättchen, Luxan C001 (Fa. ECKART), wurden in 1150 ml VE-Wasser (VE = vollentsalzt) suspendiert und unter turbulentem Rühren auf 85°C erhitzt. Der pH-Wert wurde mit verdünnter Salzsäure auf 4,2 gesenkt. Dann wurde eine Lösung bestehend aus 1,4 g $Ce(NO_3)_3$ x 6 $H_2O$ gelöst in 40mL VE-Wasser zudosiert. Gleichzeitig wurde durch Zutropfen einer 10%igen NaOH-Lösung der pH-Wert konstant gehalten. Nachdem die Lösung vollständig zugegeben wurde, wurde 1 h nachgerührt und im Anschluss daran der pH-Wert mit verdünnter Natronlauge auf 10 eingestellt. Danach wurden 5,7g Dynasylan 1146 verdünnt mit 24,3g VE-Wasser in die Suspension zugegeben und 180 min nachgerührt, dann wurde die Suspension abfiltriert und der Filterkuchen mit VE-Wasser nachgewaschen. Der Filterkuchen wurde bei 95°C unter Vakuum getrocknet. Das Pigment hatte einen theoretischen Ce-Gehalt von 0,3 Gew.-%, bezogen auf das Gesamtgewicht des Pigments.
Das Pigment wies folgende Partikelgrößenverteilung auf (MALVERN Mastersizer MS 2000): $D_{10}$ = 17,4 $\mu$m, $D_{50}$ = 35,8 $\mu$m, $D_{90}$ = 65,5 $\mu$m.

**Beispiel 2**

100 g kommerziell erhältliches silbernes Perlglanzpigment auf Basis von synthetischem Glimmer, Symic C001 (Fa. ECKART), wurden in 850 ml VE-Wasser suspendiert und unter turbulentem Rühren auf 85 °C erhitzt. Der pH-Wert wurde mit verdünnter Salzsäure auf 4,2 gesenkt. Dann wurde eine Lösung bestehend aus 0,93 g $Ce(NO_3)_3$ x 6 $H_2O$ gelöst in 40mL VE-Wasser zudosiert. Gleichzeitig wurde durch Zutropfen einer 10%igen NaOH-Lösung der pH-Wert konstant gehalten. Nachdem die Lösung vollständig zugegeben wurde, wurde 1 h nachgerührt und im Anschluss daran der pH-Wert mit verdünnter Natronlauge auf 10 eingestellt. Danach wurden 5,7g Dynasylan Hydrosil 2627 verdünnt mit 24,3g VE-Wasser in die Suspension zugegeben und 180 min nachgerührt, dann wurde die Suspension abfiltriert und der

Filterkuchen mit VE-Wasser nachgewaschen. Der Filterkuchen wurde bei 95°C unter Vakuum getrocknet. Das Pigment hatte einen theoretischen Ce-Gehalt von 0,3 Gew.-%, bezogen auf das Gesamtgewicht des Pigments.

Das Pigment wies folgende Partikelgrößenverteilung auf (MALVERN Mastersizer MS 2000): $D_{10}$ = 12,0 $\mu$m, $D_{50}$ = 23,1 $\mu$m, $D_{90}$ = 41,6 $\mu$m.

**Vergleichsbeispiel 1**

100 g kommerziell erhältliches silbernes Perlglanzpigment auf Basis von Glasplättchen, Luxan C001 (Fa. ECKART), wurden in 500 ml Isopropanol suspendiert und auf Siedetemperatur gebracht.
Unter Rühren gab man zunächst 2,0 g $H_2O$ und anschließend innerhalb einer Stunde eine Lösung aus 0,93 g $Ce(NO_3)_3$ x 6 $H_2O$ in 8 g Isopropanol hinzu. Anschließend wurde eine Lösung von 0,45 g Ethylendiamin in 3,0 g $H_2O$ hinzugegeben. Danach leitete man über einen Zeitraum von 2h 7,0 g Tetraethoxysilan und 22 g Isopropanol mit einer Dosierpumpe (Ismatec) kontinuierlich ein. Anschließend ließ man die Suspension noch 6 h lang weiter reagieren. Dann gab man 0,4 g Dynasylan AMEO und 1,3 g Dynasylan 9116 hinzu und ließ langsam abkühlen. Das Gemisch wurde über Nacht bei Raumtemperatur gerührt und am nächsten Tag abfiltriert. Der Pigmentfilterkuchen wurde anschließend bei 80°C unter Vakuum getrocknet.
Das Pigment hatte einen theoretischen Ce-Gehalt von 0,3 Gew.-% und einen $SiO_2$-Gehalt von 1,9 Gew.-%, jeweils bezogen auf das Gesamtgewicht des Pigments. Das Pigment wies folgende Partikelgrößenverteilung auf (MALVERN Mastersizer MS 2000): $D_{10}$ = 17,6 $\mu$m, $D_{50}$ = 36,0 $\mu$m, $D_{90}$ = 66,3 $\mu$m.

**Vergleichsbeispiel 2**

100 g kommerziell erhältliches silbernes Perlglanzpigment auf Basis von synthetischem Glimmer, Symic C001 (Fa. ECKART), wurden in 500 ml Isopropanol suspendiert und auf Siedetemperatur gebracht.
Unter Rühren gab man zunächst 2,0 g $H_2O$ und anschließend innerhalb einer Stunde eine Lösung aus 0,93 g $Ce(NO_3)_3$ x 6 $H_2O$ in 8 g Isopropanol hinzu. Anschließend wurde eine Lösung von 0,45 g Ethylendiamin in 3,0 g $H_2O$ hinzugegeben. Danach leitete man über einen Zeitraum von 2h 10,4 g Tetraethoxysilan und 22 g Isopropanol mit einer Dosierpumpe (Ismatec) kontinuierlich ein. Anschließend ließ man die Suspension noch 6 h lang weiter reagieren. Dann gab man 0,4 g Dynasylan AMEO und 1,3 g Dynasylan 9116 hinzu und ließ langsam abkühlen. Das Gemisch wurde über Nacht bei Raumtemperatur gerührt und am nächsten Tag abfiltriert. Der Pigmentfilterkuchen wurde anschließend bei 80°C unter Vakuum getrocknet.
Das Pigment hatte einen theoretischen Ce-Gehalt von 0,3 Gew.-% und einen $SiO_2$-Gehalt von 2,8 Gew.-%, jeweils bezogen auf das Gesamtgewicht des Pigments. Das Pigment wies folgende Partikelgrößenverteilung auf (MALVERN Mastersizer MS 2000): $D_{10}$ = 12,2 $\mu$m, $D_{50}$ = 23,4 $\mu$m, $D_{90}$ = 42,5 $\mu$m.

Vergleichsbeispiel 3

Kommerziell erhältliches wetterstabilisiertes Perlglanzpigment, Phoenix CFE 1001 (Fa. ECKART). Das Pigment wies folgende Partikelgrößenverteilung auf (MALVERN Mastersizer MS 2000): $D_{10}$ = 9,4 $\mu$m, $D_{50}$ = 20,8 $\mu$m, $D_{90}$ = 39,0 $\mu$m.

**Vergleichsbeispiel 4**

100 g kommerziell erhältliches silbernes Perlglanzpigment auf Basis von Glasplättchen, Luxan C001 (Fa. ECKART), wurden in 500 ml Isopropanol suspendiert und auf Siedetemperatur gebracht.
Unter Rühren gab man zunächst 2,0 g $H_2O$ und anschließend innerhalb einer Stunde eine Lösung aus 0,93 g $Ce(NO_3)_3$ x 6 $H_2O$ in 8 g Isopropanol hinzu. Anschließend ließ man die Suspension noch 1 h lang weiter reagieren. Das Gemisch wurde über Nacht bei Raumtemperatur gerührt und am nächsten Tag abfiltriert. Der Pigmentfilterkuchen wurde anschließend bei 80°C unter Vakuum getrocknet.

Das Pigment hatte einen theoretischen Ce-Gehalt von 0,3 Gew.-% bezogen auf das Gesamtgewicht des Pigments.
Das Pigment wies folgende Partikelgrößenverteilung auf (MALVERN Mastersizer MS 2000): $D_{10}$ = 17,5 μm, $D_{50}$ = 35,9 μm, $D_{90}$ = 65,7 μm.

## II Charakterisierung der Pigmente

### IIa Teilchengrößenmessung

Die Größenverteilungskurve der plättchenförmigen synthetischen Substrate sowie der Perlglanzpigmente wurde mit einem Gerät der Fa. Malvern (Gerät: MALVERN Mastersizer 2000) gemäß Herstellerangaben bestimmt. Hierzu wurden ca. 0,1 g des entsprechenden Substrates bzw. Pigmentes als wässrige Suspension, ohne Zusatz von Dispergierhilfsmitteln, unter ständigem Rühren mittels einer Pasteurpipette in die Probenvorbereitungszelle des Messgerätes gegeben und mehrmals vermessen. Aus den einzelnen Messergebnissen wurden die resultierenden Mittelwerte gebildet. Die Auswertung der Streulichtsignale erfolgte dabei nach der Fraunhofer Methode.

Unter der mittleren Größe $D_{50}$ wird im Rahmen dieser Erfindung der $D_{50}$-Wert der Summenhäufigkeitsverteilung der volumengemittelten Größenverteilungsfunktion, wie sie durch Laserbeugungsmethoden erhalten werden, verstanden. Der $D_{50}$-Wert gibt an, dass 50 % der nichtmetallischen plättchenförmigen synthetischen Substrate bzw. Pigmente einen Durchmesser aufweisen, der gleich oder kleiner als der angegebene Wert, beispielsweise 20 μm, ist. Entsprechend gibt der $D_{90}$-Wert an, dass 90% der Substrate bzw. Pigmente einen Durchmesser aufweisen, der gleich oder kleiner als der jeweilige Wert ist. Weiterhin gibt der $D_{10}$-Wert an, dass 10% der Substrate bzw. Pigmente einen Durchmesser aufweisen, der gleich oder kleiner als der jeweilige Werte ist.

### IIb Bestimmung der mittleren Dicke der plättchenförmigen synthetischen Substrate

Zur Bestimmung der mittleren Dicke der nichtmetallischen plättchenförmigen synthetischen Substrate wurden die Substrate oder die Pigmente 10 Gew.-%ig in einem 2K Klarlack Autoclear Plus HS, Fa. Sikkens, mit einem Hülsenpinsel eingearbeitet und mit Hilfe einer Spiralrakel (26μm Nassfilmdicke) auf eine Folie appliziert und getrocknet. Nach 24h Trocknung wurden von diesen Rakelabzügen Querschliffe angefertigt, die in rasterelektronenmikroskopisch vermessen wurden. Hierbei wurden mindestens 100 Pigmentteilchen vermessen, um eine aussagefähige Statistik zu erhalten.

### IIc Bestimmung des Ceroxidgehalts

Die Ceroxidgehalt der Pigmente wurden mittels Röntgenfluoreszenzanalyse (RFA) bestimmt.
Hierzu wurde das Pigment in eine Lithiumtetraboratglastablette eingearbeitet, in Festprobenmessbechern fixiert und daraus vermessen. Als Messgerät diente das Gerät Advantix ARL, Fa. Thermo Scientific.

## III Wetterbeständigkeit der Pigmente

### A Schwitzwassertest

Einige Pigmentproben wurden in ein Wasserlacksystem eingearbeitet und die Prüfapplikationen durch Spritzlackierung hergestellt. Der Basislack wurde mit einem handelsüblichen 1 K-Klarlack überlackiert und anschließend eingebrannt. Diese Applikationen wurden nach DIN 50 017 (Kondenswasser-Konstantklimate) geprüft. Die Haftfestigkeit wurde mittels Gitterschnitt nach DIN EN ISO 2409 sofort nach Testende im Vergleich zur unbelasteten Probe geprüft. Hierbei bedeutet Gt 0 keine Veränderung und Gt 5 eine sehr starke Veränderung.
Das Quellverhalten wurde unmittelbar nach Schwitzwasserbelastung in Anlehnung an die DIN 53230 visuell beurteilt. Hierbei bedeutet die Kennzahl 0: keine Veränderung und die Kennzahl 5: sehr starke Veränderung.
Schließlich wurde der DOI (distinctness of image) visuell beurteilt. Er kann sich im Wesentlichen aufgrund der Quellvorgänge durch Wassereinlagerung verändern.

**Tabelle 1: Schwitzwasserergebnisse**

| Probe | Glanz 20° vor SW-Test | Glanz 20° nach SW-Test | Glanzverlust | DOI | Gitterschnitt sofort | Quellung visuell |
|---|---|---|---|---|---|---|
| Beispiel 1 | 90,3 | 89,7 | <1 % | 78,2 | 0 | 0 |
| Beispiel 2 | 91,2 | 90,8 | <1 % | 80,4 | 0 | 0 |

(fortgesetzt)

| Probe | Glanz 20° vor SW-Test | Glanz 20° nach SW-Test | Glanzverlust | DOI | Gitterschnitt sofort | Quellung visuell |
|---|---|---|---|---|---|---|
| Vergleichs beispiel 1 | 94,1 | 86,6 | 8% | 70,5 | 3 | 2 |
| Vergleichs beispiel 2 | 92,5 | 90,1 | 3% | 80,2 | 1 | 1 |
| Vergleichs beispiel 3 | 91,3 | 88,1 | 4% | 77,6 | 1 | 2 |
| Vergleichs beispiel 4 | 90,9 | 30,7 | 66% | 80,8 | 3 | 4 |

Die erfindungsgemäßen Beispiele wiesen eine optimale Schwitzwasserstabiltät auf. Alle Vergleichsbeispiele zeigten eine deutlich schlechtere Haftfestigkeit (Gitterschnitt) was sich u.a. durch die Verwendung von monomeren Silansystemen erklären lässt. Da sich die Hydrolyse- und Kondensationsgeschwindigkeiten verschiedener monomerer Silane mithin deutlich unterscheiden können (bis zum 850xfachen), wie in "Hydrolysis and Condensation of organosilanes - EU 10-002/MS/fk/Sept. 97" beschrieben, ist es bei der Verwendung zweier unterschiedlicher Silane, wie in den Vergleichsbeispielen erfolgt, sehr wahrscheinlich, dass das Aminosilan deutlich eher hydrolysiert und/oder kondensiert und sich damit zuerst auf der Pigmentoberfläche festsetzen kann. Auch ist somit der Vernetzungsgrad des bereits umgesetzten Aminosilans unterschiedlich und die Oberflächenmodifizierung kann sehr inhomogen sein. Erst deutlich später kommt es zur Kondensation und damit zur Fällung des Alkylsilanes, welches auf diese Weise einen Teil der vorgelegten Vernetzungsgruppen aus dem Aminosilan überdecken kann, und diese nicht mehr für die Anbindung an das Lacksystem zur Verfügung stehen. Auch dürfte eine Belegung mit Alkylsilan sowohl aufgrund der Vorbelegung mit bereits umgesetzten Aminosilan als auch aufgrund sterischer Hinderung durch die Alkylgruppe nicht optimal an der beschichteten Pigmentoberfläche anbinden können, was die stärkere Quellung der Lackschicht und damit ungünstigere Zwischenhaftung erklären könnte.

Speziell Pigment aus Vergleichsbeispiel 4 besitzt keinerlei organisch-chemische Kompatibilisierungsschicht, worin die unzureichende Haftfestigkeit als auch die Quellung der umgebenden Bindemittelschicht (Glanz nach SW-Test) erklärt werden kann.

**B WOM-Test**

Die Pigmentproben wurden in ein Wasserlacksystem eingearbeitet und die Prüfapplikationen durch Spritzlackierung hergestellt. Der Basislack wurde mit einem handelsüblichen Klarlack überlackiert und anschließend eingebrannt. Die beschleunigte Bewitterungsprüfung erfolgte nach SAE 2527 in einem Q-Sun Xe 3 HS (Fa. Q-Lab) Xenon-Testgerät. Die Bestimmung der $\Delta E^*$-Werte sowie die Einstufung nach der Grauskala erfolgten jeweils relativ zur entsprechenden unbelasteten Probe.

| | Glanzverlust 20° nach 3000 h |
|---|---|
| Beispiel 1 | 26% |
| Vergleichsbeispiel 1 | 34% |

Der Glanzabbau ist beim Vergleichsbeispiel deutlich stärker ausgeprägt.

**C UV-Beständigkeit an Rakelabzügen**

Dieser Test wurde in Anlehnung an den in der EP 0 870 730 beschrieben UV-Schnelltest zur Bestimmung der photochemischen UV-Aktivität von $TiO_2$-Pigmenten durchgeführt.

Hierzu wurden 1,0 g des Perlglanzpigmentes in 9,0 g eines doppelbindungsreichen melaminhaltigen Lackes eindispergiert. Es wurden Rakelabzüge auf kartoniertem Papier angefertigt und diese bei Raumtemperatur getrocknet. Die Rakelabzüge wurden geteilt und jeweils einer der beiden Abschnitte als unbelastetes Vergleichsmuster im Dunkeln gelagert. Anschließend wurden die Proben 150 min lang in einem QUV-Gerät der Fa. Q-Panel mit UV-haltigem Licht (UVA-340 Lampe, Bestrahlungsstärke 1,0 $W/m^2/nm$) bestrahlt. Unmittelbar nach dem Testende wurden mit einem Farbmessgerät CM-508i der Fa. Minolta Farbwerte der belasteten Prüflinge relativ zum jeweiligen Rückstellmuster ermittelt. Die resultierenden ΔE*-Werte sind, nach der Hunter-L*a*b*-Formel berechnet, in Tab.2 dargestellt.

Im Test wird im Wesentlichen eine graublaue Verfärbung der $TiO_2$-Schicht des Perlglanzpigmentes in den Rakelabzügen aufgrund von unter UV-Lichteinfluß gebildeten Ti(III)-Zentren beobachtet. Bedingung hierfür ist, dass das Elektronenloch das $TiO_2$ räumlich verlassen hat und - etwa durch Reaktion mit olefinischen Doppelbindungen des Bindemittels - nicht unmittelbar wieder mit dem verbleibenden Elektron rekombinieren kann. Da eine melaminhaltige Lackschicht die Diffusion von Wasser(dampf) und Sauerstoff an die Pigmentoberfläche signifikant verlangsamt, findet eine Reoxidation der Titan(III)-Zentren deutlich verzögert statt, so dass die Vergrauung gemessen und der ΔE-Wert als Maß für die UV-Stabilität der Pigmente herangezogen werden kann. Ein größerer ΔE*-Zahlenwert der belasteten Probe relativ zum unbelasteten Rückstellmuster bedeutet somit eine geringere UV-Stabilität des untersuchten Pigments.

**Tabelle 2: UV-Rakeltestergebnisse**

| Probe | Fällungsmilieu | Schicht 1 (Ce-Gehalt theor.) | Schicht 2 ($SiO_2$-gehalt theor.) | ΔE* |
|---|---|---|---|---|
| Beispiel 1 | wässrig | 0,3 % Ce | ---- | 1,01 |
| Beispiel 2 | wässrig | 0,3 % Ce | ---- | 0,97 |
| Vergleichsbeispiel 1 | alkoholisch | 0,3 % Ce | 1,9 % SiO2 | 2,55 |
| Vergleichsbeispiel 2 | alkoholisch | 0,3 % Ce | 2,8 % SiO2 | 1,70 |
| Vergleichsbeispiel 3 | alkoholisch | 0,4 % Ce | 2,8 % SiO2 | 2,01 |
| Vergleichsbeispiel 4 | alkoholisch | 0,3 % Ce | ---- | 2,54 |

Alle Vergleichsbeispiele wiesen eine deutlich stärkere Farbveränderung (ΔE*) nach entsprechender Belichtung auf. Auch dieser Einfluss kann mit der unzureichenden Kompatibilisierung der Pigmente für das entsprechende Lacksystem erklärt werden. Eine zusätzliche $SiO_2$-Schicht brachte keine Verbesserung in der UV-Stabilität. Die Verwendung oligomerer Silansysteme zeigt sich auch hier vorteilhaft.

**D Farbtonkonstanz und optische Eigenschaften im Vergleich zum Ausgangsmaterial**

Anhand von Rakelabzügen der jeweiligen Pigmente in einem konventionellen Nitrocelluloselack (Dr. Renger Erco Bronzemischlack 2615e; Fa. Morton, Pigmentierungshöhe von 6 Gew.-%, bezogen auf das Gesamtgewichtes des Nasslacks) auf Schwarz-Weiß-Deckungskarten (Byko-Chart 2853, Fa. Byk Gardner) wurden eventuell auftretende Farbtonveränderungen visuell begutachtet. Hierbei wurde festgestellt, dass visuell bei den erfindungsgemäßen Perlglanzpigmenten kein Unterschied zum Ausgangsmaterial zu erkennen ist. Dagegen zeigten Rakelabzüge der Pigmente der Vergleichsbeispiele in Bezug auf das bei den Vergleichsbeispielen eingesetzte Ausgangsmaterial ein körnigeres optisches Erscheinungsbild.

## Patentansprüche

1. Wetterstabiles Perlglanzpigment, das aus einem mit einem oder mehreren hochbrechenden Metalloxid(en) beschichteten plättchenförmigem Substrat, das aus der Gruppe, die aus synthetischen Glimmerplättchen, Glasplättchen, $SiO_2$-Plättchen, $Al_2O_3$-Plättchen, synthetischen Böhmitplättchen, BiOCl-Plättchen und deren Mischungen besteht, ausgewählt wird und einer Deckschicht besteht, wobei die Deckschicht aus folgenden Schichten besteht:

   a) einer Cer-haltigen Schicht, welche aus Ceroxid und/ oder Cerhydroxid und/ oder Ceroxidhydrat besteht und die Cer-haltige Schicht direkt auf der hochbrechenden Metalloxidschicht aufgebracht ist,
   b) einer organisch-chemischen Kompatibilisierungsschicht, welche die Reaktionsprodukte aus oligomeren Silanen enthält oder daraus besteht, wobei die oligomeren Silane eine oder mehrere Aminogruppen aufweisen und die organisch-chemische Kompatibilisierungsschicht direkt auf der Cer-haltigen Schicht a) aufgebracht ist.

2. Wetterstabiles Perlglanzpigment nach Anspruch 1,
   **dadurch gekennzeichnet,**
   **dass** das plättchenförmige Substrat aus der Gruppe, die aus synthetischen Glimmerplättchen, Glasplättchen, $Al_2O_3$-Plättchen und deren Mischungen besteht, ausgewählt wird.

**3.** Wetterstabiles Perlglanzpigment nach einem der vorstehenden Ansprüche, **dadurch gekennzeichnet,** **dass** die Schicht a) in Mengen aus einem Bereich von 0,05 bis unter 0,9 Gew.-%, bezogen auf das Gesamtgewicht des Perlglanzpigments und berechnet als elementares Cer, vorhanden ist.

**4.** Wetterstabiles Perlglanzpigment nach einem der vorstehenden Ansprüche, **dadurch gekennzeichnet,** **dass** die oligomeren Silane eine odere mehrere Alkylgruppen von 1 bis 18 C-Atome aufweisen.

**5.** Wetterstabiles Perlglanzpigment nach einem der vorstehenden Ansprüche, **dadurch gekennzeichnet,** **dass** Anteil der Deckschicht am gesamten Perlglanzpigment 0,3 bis 2,5 Gew.-%, bezogen auf das Gesamtgewicht des Perlglanzpigments beträgt.

**6.** Wetterstabiles Perlglanzpigment nach einem der vorstehenden Ansprüche, **dadurch gekennzeichnet,** **dass** die Beschichtung des Perlglanzpigmentes mit der gesamten Deckschicht in wässrigem Milieu erfolgt ist.

**7.** Wetterstabiles Perlglanzpigment nach einem der vorstehenden Ansprüche, **dadurch gekennzeichnet,** **dass** das Substrat mit einer (Zahl: 1) hochbrechenden Metalloxidschicht bestehend aus der Gruppe $TiO_2$, $Fe_2O_3$, $TiFe_2O_5$, $Fe_2Ti_3O_9$, $FeTiO_3$ und Mischungen davon beschichtet ist.

**8.** Wetterstabiles Perlglanzpigment nach einem der vorstehenden Ansprüche, **dadurch gekennzeichnet,** **dass** das wetterstabile Perlglanzpigment die Kennzahlen $D_{10}$, $D_{50}$, $D_{90}$ aus der Summenhäufigkeitsverteilung der volumengemittelten Größenverteilungsfunktion mit einem Span $\Delta D$ von 0,7 - 1,4 aufweist, wobei der Span $\Delta D$ gemäß Formel (I) berechnet ist:

$$\Delta D = (D_{90} - D_{10}) / D_{50} \qquad \text{(I)}$$

**9.** Wetterstabiles Perlglanzpigment nach Anspruch 8, **dadurch gekennzeichnet,** **dass** das Perlglanzpigment einen Span $\Delta D$ von 0,75 - 1,3 aufweist.

**10.** Verfahren zur Herstellung des wetterstabilen Perlglanzpigmentes nach einem der vorstehenden Ansprüche, **dadurch gekennzeichnet,** **dass** es folgende Schritte umfasst:

a) Optional Klassieren des plättchenförmigen Substrates unter Erhalt von Substraten, welche die Kennzahlen $D_{10}$, $D_{50}$, $D_{90}$ aus der Summenhäufigkeitsverteilung der volumengemittelten Größenverteilungsfunktion mit einem Span $\Delta D$ von 0,7 - 1,4 aufweisen,
b) Suspendieren eines plättchenförmigen Substrates, optional aus Schritt a), in wässriger Lösung und Beschichten des plättchenförmigen Substrates mit einem oder mehreren hochbrechenden Metalloxid(en) unter Erhalt von Perlglanzpigmenten,
c) Beschichten der Perlglanzpigmente aus Schritt b) in wässriger Lösung mit einem Cer-salz oder einer hydrolysierbaren Cer-metallorganischen Verbindung unter Erhalt einer Schicht, die aus der Gruppe, die aus Cerhydroxid, Ceroxidhydrat und Mischungen davon besteht, ausgewählt wird,
d) Beschichten der Perlglanzpigmente aus Schritt c) in wässriger Lösung mit oligomeren Silanen,
e) Abtrennen der beschichteten Perlglanzpigmente, optional Waschen mit vollentsalztem Wasser, und Trocknen bei einer Temperatur von bei 80° bis 160°C.

**11.** Verwendung der wetterstabilen Perlglanzpigmente nach einem der Ansprüche 1 bis 9 zur Pigmentierung von Lacken, Druckfarben, Kunststoffen und Kosmetika.

## Claims

1. Weather-resistant pearlescent pigment, comprising a platelet-shaped substrate coated with one or more highly refractive metal oxides, selected from the group comprising synthetic mica platelets, glass platelets, $SiO_2$ platelets, $Al_2O_3$ platelets, synthetic boehmite platelets, BiOCl platelets and mixtures thereof, and a top coat, and the top coat comprises the following layers:

   a) a cerium-containing layer comprising cerium oxide and/or cerium hydroxide and/or hydrated cerium oxide and the cerium-containing layer is applied directly to the highly refractive metal oxide layer,
   b) an organic-chemical compatibilising layer, containing or comprising the reaction products of oligomeric silanes, and the oligomeric silanes have one or more amino groups and the organic-chemical compatibilising layer is applied directly to the cerium-containing layer a).

2. Weather-resistant pearlescent pigment as claimed in claim 1,
   **characterised in that**
   the platelet-shaped substrate is selected from the group comprising synthetic mica platelets, glass platelets, $Al_2O_3$ platelets and mixtures thereof.

3. Weather-resistant pearlescent pigment as claimed in one of the preceding claims,
   **characterised in that**
   layer a) is present in quantities in a range of from 0.05 to less than 0.9 % by weight, relative to the total weight of the pearlescent pigment and calculated as elemental cerium.

4. Weather-resistant pearlescent pigment as claimed in one of the preceding claims,
   **characterised in that**
   the oligomeric silanes have one or more alkyls groups of 1 to 18 C atoms.

5. Weather-resistant pearlescent pigment as claimed in one of the preceding claims,
   **characterised in that**
   the proportion of top coat on all the pearlescent pigment is 0.3 to 2.5 % by weight, relative to the total weight of the pearlescent pigment.

6. Weather-resistant pearlescent pigment as claimed in one of the preceding claims,
   **characterised in that**
   coating of the pearlescent pigment with the entire top coat takes place in aqueous medium.

7. Weather-resistant pearlescent pigment as claimed in one of the preceding claims,
   **characterised in that**
   the substrate is coated with a (one) highly refractive metal oxide layer from the group comprising $TiO_2$, $Fe_2O_3$, $TiFe_2O_5$, $Fe_2Ti_3O_9$, $FeTiO_3$ and mixtures thereof.

8. Weather-resistant pearlescent pigment as claimed in one of the preceding claims,
   **characterised in that**
   the weather-resistant pearlescent pigment has $D_{10}$, $D_{50}$, $D_{90}$ values from the cumulative frequency distribution of the volume-averaged size distribution function with a span $\Delta D$ of 0.7 - 1.4, and the span $\Delta D$ is calculated in accordance with formula (I):

$$\Delta D = (D_{90} - D_{10})/D_{50} \qquad (I).$$

9. Weather-resistant pearlescent pigment as claimed in claim 8,
   **characterised in that**
   the pearlescent pigment has a span $\Delta D$ of 0.75 - 1.3.

10. Method of producing the weather-resistant pearlescent pigment as claimed in one of the preceding claims,
    **characterised in that**
    it comprises the following steps:

a) optionally classifying the platelet-shaped substrate to obtain substrates having $D_{10}$, $D_{50}$, $D_{90}$ values from the cumulative frequency distribution of the volume-averaged size distribution function with a span $\Delta D$ of 0.7 - 1.4,
b) suspending a platelet-shaped substrate, optionally from step a), in aqueous solution and coating the platelet-shaped substrate with one or more highly refractive metal oxides to obtain pearlescent pigments,
c) coating the pearlescent pigments from step b) in aqueous solution with cerium salt or a hydrolysable cerium metal-organic compound to obtain a layer selected from the group comprising cerium oxide, hydrated cerium oxide and mixtures thereof,
d) coating the pearlescent pigments from step c) in aqueous solution with oligomeric silanes,
e) separating the coated pearlescent pigments, optionally washing with demineralised water, and drying at a temperature of 80° to 160°C.

**11.** Use of the weather-resistant pearlescent pigments as claimed in one of claims 1 to 9 as a pigment for varnishes, printing inks, plastics and cosmetics.

**Revendications**

**1.** Pigment nacré stable aux intempéries, qui est constitué d'un substrat lamellaire revêtu d'un ou plusieurs oxydes métalliques à grand indice de réfraction, qui est choisi dans le groupe consistant en les lamelles de mica synthétiques, les lamelles de verre, les lamelles de $SiO_2$, les lamelles d'$Al_2O_3$, les lamelles de böhmite synthétiques, les lamelles de BiOCl et les mélanges de celles-ci, et d'une couche de couverture, la couche de couverture étant constituée des couches suivantes :

a) une couche contenant du cérium, qui est constituée d'oxyde de cérium et/ou d'hydroxyde de cérium et/ou d'oxyde de cérium hydraté, la couche contenant du cérium étant directement appliquée sur la couche d'oxyde métallique à grand indice de réfraction,
b) une couche de compatibilisation obtenue par chimie organique, qui contient le produit d'une réaction de silanes oligomères ou en est constituée, les silanes oligomères comprenant un ou plusieurs groupes amino, et la couche de compatibilisation obtenue par chimie organique étant directement appliquée sur la couche contenant du cérium a).

**2.** Pigment nacré stable aux intempéries selon la revendication 1, **caractérisé en ce que** le substrat lamellaire est choisi dans le groupe constitué de lamelles de mica synthétiques, de lamelles de verre, de lamelles d'$Al_2O_3$ et de mélanges de celles-ci.

**3.** Pigment nacré stable aux intempéries selon l'une des revendications précédentes, **caractérisé en ce que** la couche a) est présente en des quantités comprises dans la plage de 0,05 à moins de 0,9 % en poids, par rapport au poids total du pigment nacré, et exprimées en cérium élémentaire.

**4.** Pigment nacré stable aux intempéries selon l'une des revendications précédentes, **caractérisé en ce que** les silanes oligomères comprennent un ou plusieurs groupes alkyle ayant 1 à 18 atomes de carbone.

**5.** Pigment nacré stable aux intempéries selon l'une des revendications précédentes, **caractérisé en ce que** la proportion de la couche de couverture, par rapport au pigment nacré total, est de 0,3 à 2,5 % en poids, par rapport au poids total du pigment nacré.

**6.** Pigment nacré stable aux intempéries selon l'une des revendications précédentes, **caractérisé en ce que** le revêtement du pigment nacré par la couche de couverture totale a lieu en milieu aqueux.

**7.** Pigment nacré stable aux intempéries selon l'une des revendications précédentes, **caractérisé en ce que** le substrat est revêtu d'une (chiffre 1) couche d'oxyde métallique à grand indice de réfraction, constituée du groupe $TiO_2$, $Fe_2O_3$, $TiFe_2O_5$, $Fe_2Ti_3O_9$, $FeTiO_3$ et le mélange de ceux-ci.

**8.** Pigment nacré stable aux intempéries selon l'une des revendications précédentes, **caractérisé en ce que** le pigment nacré stable aux intempéries présente les caractéristiques $D_{10}$, $D_{50}$, $D_{90}$ de la distribution de fréquences cumulées de la fonction de répartition dimensionnelle moyennée en volume, avec un écart $\Delta D$ de 0,7-1,4, l'écart $\Delta D$ étant calculé selon la formule (I)

$$\Delta D = (D_{90}-D_{10})/D_{5}0 \quad (I)$$

**9.** Pigment nacré stable aux intempéries selon la revendication 8, **caractérisé en ce que** le pigment nacré présente un écart ΔD de 0,75-1,3.

**10.** Procédé de fabrication du pigment nacré stable aux intempéries selon l'une des revendications précédentes, **caractérisé en ce qu'**il comprend les étapes suivantes :

a) en option, classification du substrat lamellaire, avec obtention de substrats qui présentent les caractéristiques $D_{10}$, $D_{50}$, $D_{90}$ de la distribution de fréquences cumulées de la fonction de répartition dimensionnelle moyennée en volume avec un écart ΔD de 0,7 à 1,4,
b) mise en suspension d'un substrat lamellaire, en option de l'étape a), dans une solution aqueuse, et application sur le substrat lamellaire d'un ou plusieurs oxydes métalliques à grand indice de réfraction, avec obtention de pigments nacrés,
c) application sur les pigments nacrés de l'étape b) en solution aqueuse d'un sel de cérium ou d'un composé organométallique de cérium hydrolysable, avec obtention d'une couche qui est choisie dans le groupe consistant en l'hydroxyde de cérium, l'oxyde de cérium hydraté et le mélange de ceux-ci,
d) application sur les pigments nacrés de l'étape c) en solution aqueuse de silanes oligomères,
e) séparation des pigments nacrés revêtus, en option lavage avec de l'eau entièrement déminéralisée, et séchage à une température de 80 à 160°C.

**11.** Utilisation des pigments nacrés stables aux intempéries selon l'une des revendications 1 à 9 pour pigmenter des vernis, des encres d'imprimerie, des matières plastiques et des produits cosmétiques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0632109 A1
- EP 0888410 B1
- EP 1682622 B1
- EP 0881998 B1
- EP 1727864 A1
- EP 1084198 B1
- DE 102006009129 A1
- DE 102006009130 A1
- DE 102004041586 A1
- DE 19639783 A1
- EP 0289240 A1
- WO 2004056716 A1
- WO 2005063637 A1
- EP 1980594 B1
- EP 2217664 B1
- EP 0675128 A
- EP 0716127 A
- EP 0716128 A
- EP 0870730 A


### In der Beschreibung aufgeführte Nicht-Patentliteratur


- **M. JÄGER ; U. SCHMIDT.** Die Barriere macht den Unterschied. *Farbe und Lack,* August 2007, 20-25